# EUROPEAN PATENT SPECIFICATION

(11) **EP 0 421 668 B1**
(45) Date of publication and mention of the grant of the patent: **04.01.1995**
(21) Application number: 90310576.5
(22) Date of filing: 27.09.1990
(51) Int. Cl.: C07D 487/08, A61K 31/465, A61K 31/40

(54) **1,4-Dihydro-4-oxo-3-quinolone derivatives as selectively toxic mammalian antibacterial agents**
1,4-Dihydro-4-oxo-3-chinolinderivate als antibakterielle Wirkstoffe für Säugetieren mit selektiver Toxizität
Dérivés de dihydro-1,4-oxo-quinoléine-3-one comme agents antibactériens pour mammifères avec une toxicité sélective

(30) Priority: 06.10.1989 WO PCT/US89/04438
(43) Date of publication of application: 10.04.1991
(73) Proprietor: PFIZER INC., New York, N.Y. 10017 (US)
(72) Inventor: McGuirk, Paul R., Ledyard, Connecticut (US)
(74) Representative: Moore, James William, Dr.

(56) References cited:
- EP-A- 0 159 174
- EP-A- 0 230 274
- EP-A- 0 241 206

## Description

The present invention relates to new 1,4-dihydro-4-oxo-3-quinoline derivatives that are antibacterial agents suitable for the treatment of bacterial and mycoplasma infections in mammals, including humans. The compounds of the invention exhibit unexpectedly favorable selectivity against procaryotic cells, as measured by their activity against procaryotic DNA gyrase versus mammalian topoisomerase II.

United States Patents 4,775,668 and 4,861,779 refer to antibacterial compounds having the formula 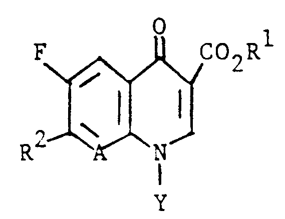
wherein R¹ is hydrogen, a pharmaceutically acceptable cation, or alkyl; A is CH, CF, CCl or N; Y is alkyl, haloalkyl, cyclopropyl, vinyl, methoxy, N-methylamino, p-flurophenyl, p-hydroxyphenyl or p-aminophenyl; or A is carbon and is taken together with Y and the carbon and nitrogen to which A and Y are attached to form a five to seven membered ring which is optionally substituted; and R² is a bridged-diazabicycloalkyl group.

Derwent anonymous research disclosure no. 88-287037 refers to compounds of the formula 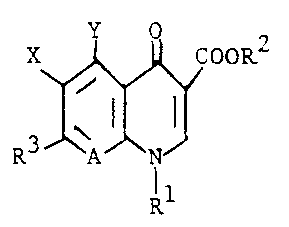
where R¹ is (C₁-C₃)alkyl, cyclopropyl, vinyl, hydroxyethyl, fluoroethyl, methoxy, amino, methylamino, dimethylamino, ethylamino, phenyl, 4-fluorophenyl or 2,4-difluorophenyl; R² is hydrogen, (C₁-C₄)alkyl or 5-methyl-2-oxo-1,3-dioxol-4-ylmethyl; R³ is methyl or one of eleven cyclic amino groups wherein one of said eleven cyclic amino groups is 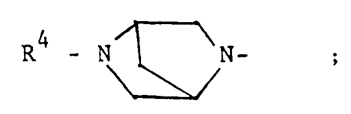
R⁴ is hydrogen, (C₁-C₄) alkyl, hydroxyethyl, allyl, propargyl, CH₂COCH₃, phenacyl, CHO, SCFCl₂, SO₂CFCl₂, SCOOMe, benzyl, 4-aminobenzyl or 5-methyl-2-oxo-1,3-dioxo-4-ylmethyl; R⁵ is hydrogen or methyl; R⁶ is hydrogen, (C₁-C₄) alkyl, phenyl or CH₂OCH₂Ph; R⁷ is hydrogen, amino, methylamino, ethylamino, aminomethyl, methylaminomethyl, ethylaminomethyl, dimethylaminomethyl, hydroxy or hydroxymethyl; R⁸ is methyl, ethyl or chloro; X is fluoro, chloro or nitro; Y is hydrogen, fluoro or amino; A is nitrogen or CR⁹; and R⁹ is hydrogen, alkoxy, (C₁-C₃)alkylthio, halogen, methyl or nitro; or R¹ and R⁹ are OCH₂CH(Me), SCH₂CH(Me), COCH₂CH(Me) or CH₂CH₂CH(Me). This broad generic disclosure includes several compounds of the present invention but does not teach or suggest their surprising and favorable selectivity as antibacterial agents against procaryotic versus eucaryotic cells.

The present invention relates to compounds of the formula 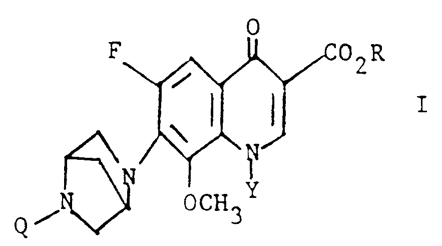
wherein Q is methyl, ethyl, hydroxyethyl or hydrogen;
R is hydrogen, (C₁-C₃) alkyl, benzyl, (C₁-C₃) alkanoyloxymethyl, (C₁-C₃)alkanoyloxyethyl, benzoyloxymethyl, benzoyloxyethyl, or 5-[(C₁-C₃)alkyl]-2-oxo-1,3-dioxolen-4-ylmethyl; and
Y is cyclopropyl or substituted cyclopropyl, wherein said substituted cyclopropyl is substituted with one to three substituents independently selected from the group consisting of (C₁-C₃)alkyl, halo hydroxy, or (C₁-C₃)alkoxy;
and the pharmaceutically acceptable salts and hydrates of such compounds.

As used herein, unless indicated otherwise, "halo" includes fluoro, chloro, bromo and iodo.

Examples of pharmaceutically acceptable salts of the compounds of formula I are the acid addition salts of acetic, lactic, succinic, maleic, tartaric, citric, gluconic, ascorbic, benzoic, methanesulfonic, cinnamic, fumaric, phosphoric, hydrochloric, hydrobromic, hydroiodic, and sulfonic acids, and the cationic salts of alkali metals such as sodium or potassium, alkaline earth metals such as magnesium or calcium, ammonium, and organic amines such as diethanolamine and N-methylglucamine.

The term "pharmaceutically acceptable salts and hydrates", as used herein, includes pharmaceutically acceptable salts, hydrates, pharmaceutically acceptable salts of hydrates, and hydrates of such salts.

The present invention also relates to a pharmaceutical composition comprising an antibacterially effective amount of a compound of the formula I, or a pharmaceutically acceptable salt thereof, and a pharmaceutically acceptable carrier.

Compounds of the formula I wherein R is (C₁-C₃)alkyl, benzyl, (C₁-C₃)alkanoyloxymethyl, (C₁-C₃)alkanoyloxyethyl, (C₁-C₃)benzoyloxymethyl, (C₁-C₃) benzoyloxyethyl or 5-[(C₁-C₃)alkyl]-2-oxo-1,3-dioxolen-4-ylmethyl, are prodrugs of compounds of the formula I wherein R is hydrogen.

The compounds of the formula I may have chiral centers and may exist in several stereoisomeric forms. This invention includes all stereoisomers of the compounds of formula I, and mixtures thereof.

Preferred compounds of the invention are 1-cyclopropyl-6-fluoro-8-methoxy-7- (1S,4S)-5-methyl-2,5-diazabicyclo[2.2.1]hept-2-yl -1,4-dihydro-4-oxo-3-quinoline carboxylic acid and the pharmaceutically acceptable salts and hydrates thereof.

Compounds of the formula I may be prepared as illustrated in the following reaction scheme. 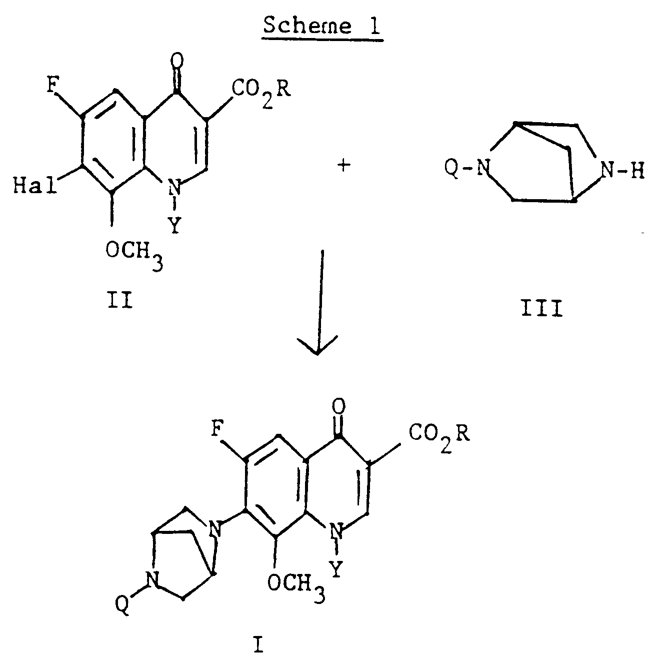
Referring to the above reaction scheme, compounds of the formula I, wherein Q is hydrogen and R and Y are as defined above, may be prepared by reacting a compound of the formula II, wherein R and Y are as defined above and Hal is chloro, bromo, fluoro or iodo, with a compound of the formula III.

The reaction may be performed in the absence of a solvent or in the presence of a polar, reaction inert solvent such as acetonitrile, tetrahydrofuran, ethanol, chloroform, dimethylformamide, pyridine, or water, or mixtures thereof. The reaction is preferably carried out in the presence of a base such as an alkali metal or alkaline earth metal carbonate or bicarbonate, or a secondary or tertiary amine such as triethylamine, pyridine or picoline. The preferred reaction temperature is from about 60 to about 100°C, but temperatures from about 10 to about 150°C are generally suitable.

The starting materials of formula II are known in the art, e.g. as disclosed in European Patent Application 0230295A2.

Compounds of the formula III, wherein Q is hydrogen, may be made as described in U.S. Patent 3,947,445 and in J. Org. Chem., 31, 1059 (1966).

Compounds of the formula I wherein Q is methyl may be prepared from the corresponding compounds of the formula I wherein Q is hydrogen by conventional methylation procedurs. For example, one standard methylation procedure known in the art involves the use of formic acid and paraformaldehyde. According to this procedure, a mixture of an alkali or alkaline earth metal formate (preferably sodium formate), formic acid (preferably 87% formic acid), formalin (preferably 37% formalin), and a compound of the formula I wherein Q is hydrogen are reacted to yield a compound of the formula I wherein Q is methyl. The reaction is typically carried out by stirring the reactants for approximately 0.5 to 24 hours, preferably about 2 hours, at a temperature of from about 80 to about 150°C, preferably from 100 to 120°C. The compounds of formula I so produced can be isolated by conventional purification techniques.

Compounds of the formula I wherein Q is ethyl or hydroxyethyl may be prepared by reacting a compound of the formula I wherein Q is hydrogen with, respectively, ethyl iodide or 2-bromoethanol. This reaction is generally carried out in a reaction inert solvent such as tetrahydrofuran (THF) or dimethylformamide (DMF), and in the presence of an organic base such as triethylamine, at a temperature of from about 50 to 150°C, preferably about 100°C, for approximately 24 hours. The compound of formula I so produced can be isolated by conventional purification techniques.

In each of the reactions described above, pressure is not critical. Pressures from about 0.5 to about 3 atmospheres are generally suitable, and ambient pressure is preferred as a matter of convenience.

The pharmaceutically acceptable acid addition salts of compounds the formula I may be prepared in a conventional manner by treating a solution or suspension of the free base of the formula I with about one chemical equivalent of a pharmaceutically acceptable acid. Conventional concentration and recrystallization techniques are employed in isolating the salts.

The pharmaceutically acceptable cationic salts of compounds of the formula I may be prepared by conventional methods from the corresponding acids, e.g. by reaction with about one equimolar amount of a base.

The novel compounds of the formula I and the pharmaceutically acceptable acid addition salts thereof are useful in the treatment of bacterial infections of broad spectrum, particularly in the treatment of gram-positive bacterial infections.

The compounds of the invention may be administered alone, but will generally be administered in admixture with a pharmaceutical carrier selected with regard to the intended route of administration and standard pharmaceutical practice. For example, they can be administered orally or in the form of tablets containing such excipients as starch or lactose, or in capsules either alone or in admixture with excipients, or in the form of elixirs or suspensions containing flavoring or coloring agents. In the case of animals, they are advantageously contained in an animal feed or drinking water in a concentration of about 5 to about 5000 ppm, preferably about 25 to about 500 ppm. They can be injected parenterally, for example, intramuscularly, intravenously or subcutaneously. For parenteral administration, they are best used in the form of a sterile aqueous solution which can contain other solutes, for example, enough salt or glucose to make the solution isotonic. In the case of animals, compounds can be administered intramuscularly or subcutaneously at dosage levels of about 0.1 to about 50 mg/kg/day, preferably about 0.2 to about 10 mg/kg/day given in a single daily dose or up to 4 divided doses.

The compounds of the invention can be administered to humans for the treatment of bacterial diseases by either the oral or parenteral routes and may be administered orally at dosage levels of about 0.1 to about 500 mg/kg, advantageously about 0.5 to about 50 mg/kg/day given in a single dose or up to 4 divided doses. For intramuscular or intravenous administration, dosage levels are about 0.1 to about 200 mg/kg/day, preferably about 0.5 to about 50 mg/kg/day. While intramuscularly administration may be a single dose or up to 4 divided doses, intravenous administration can include a continuous drip. Variations will necessarily occur depending on the weight and condition of the subject being treated and the particular route of administration chosen as will be known to those skilled in the art.

The antibacterial activity of the compounds of the present invention may be determined by testing according to the Steer's replicator techniques, which is a standard in vitro bacterial testing method described by E. Steers et al., Antibiotics and Chemotherapy, 9, 307 (1959). The selective antibacterial activity of the compounds of the formula I against procaryotic versus eucaryotic cells may be determined by comparing their activity against procaryotic DNA-gyrase versus mammalian topoisomerase II according to the procedure of Barrett et al., Antimicrobial Agents and Chemotherapy, 33 (10) (October, 1989).

The following examples serve to illustrate but not limit the present invention.

### Example 1

### a. Ethyl 3-methoxy-2,4,5-trifluorobenzoylacetate

Dianion of monoethylmalonate: To a solution of monoethylmalonic acid (9.8 g, 73 mmol) in anhydrous tetrahydrofuran (350 mL) at -78°C was added n-butyllithium (50 mL, 80 mmol, 1.6 M, 1.1 equiv). The reaction temperature was then raised to -5°C and additional n-butyllithium was added (50 mL, 80 mmol, 1.6 M, 1.1 equiv.) dropwise. The reaction mixture was allowed to stir for an additional hour at -5°C and was then cooled to 78°C.

Addition of acid chloride: 3-Methoxy-2,4,5-trifluorobenzoyl chloride (6.38 g, 24 mmol), prepared from 3-methoxy-2,4,5-trifluorobenzoic acid (5.0 g, 24 mmol) and excess thionyl chloride at reflux for 2 hours followed by removal of excess thionyl chloride in vacuo, was added dropwise as a solution in anhydrous tetrahydrofuran (50 mL) to the dianion of monoethylmalonate at -78°C. The reaction mixture was allowed to warm to room temperature for 2 hours and was quenched by pouring into 1N hydrochloric acid. The aqueous layer was extracted with diethyl ether (3 x 150 mL) and the combined organic layers were washed with saturated aqueous sodium bicarbonate and water, dried over anhydrous magnesium sulfate, filtered and concentrated in vacuo to afford 5.15 g, 76% yield of a white solid after recrystallization from hexanes, m.p. 42-44°C.

Anal.: Calcd. for C₁₂H₁₁F₃O₄: C, 52.20; H, 3.90. Found: C, 52.18; H, 3.85.

MS (low resolution) M+ = 276.

### b. Ethyl 2-(3-methoxy-2,4,5-triflurobenzoyl)-3-ethoxyacrylate

A mixture of ethyl-3-methoxy-2,4,5-trifluorobenzoylacetate (500 mg, 1.8 mmol), ethyl orthoformate (0.45 mL, 2.72 mmol) and acetic anhydride (460 mg, 4.5 mmol, 2.5 eq) was stirred at 110°C for 12 hours and the excess reagents were removed by distillation under high vacuum (0.1 mmHg) to give 590 mg of a yellow-orange oil. This material was used directly in the next step without further purification or characterization.

### c. Ethyl 2-(3-methoxy-2,4,5-trifluorobenzoyl)-3-cyclopropylaminoacrylate

To a solution of ethyl 2-(3-methoxy-2,4,5-triflurobenzoyl)-3-ethoxyacrylate (590 mg, 1.8 mmol) in methylene chloride (15 mL) at 0°C was added a solution of cyclopropylamine (114 mg, 1.98 mmol, 1.2 equiv) in methylene chloride (5 mL). The reaction mixture was warmed to room temperature and allowed to stir for 2 hours. The solvent was removed in vacuo and the crude concentrate was purified by flash chromatography on silica gel (methylene chloride/ethyl acetate 50:1 v/v) to provide 438 mg, 62% yield over two steps, of a viscous yellow oil.

### d. Ethyl 8-methoxy-1-cyclopropyl-6,7-difluoro-1,4-dihydro-4-oxo-3-quinolinecarboxylate

To a solution of ethyl 2-(3-methoxy-2,4,5-trifluorobenzoyl)-3-cyclopropylaminoacrylate (440 mg, 1.29 mmol) in anhydrous tetrahydrofuran (15 mL) at 0°C was added sodium hydride (62 mg, 1.5 mmol, 1.1 equiv, 60% in mineral oil). The reaction mixture was allowed to stir at 50°C for 12 hours. The mixture was cooled to room temperature and filtered. The mother liquor was concentrated and a white precipitate formed. The material was collected by suction filtration and air-dried to give 312 mg, 75% yield of a pure white solid, m.p. 178-181°C.

### e. 1-Cyclopropyl-6,7-difluoro-8-methoxy-1,4-dihydro-4-oxo-3-quinolinecarboxylic acid

A mixture of ethyl-1-cyclopropyl-6,7-difluoro-8-methoxy-1,4-dihydro-4-oxo-3-quinoline carboxylic acid (500 mg, 1.55 mmol), glacial acetic acid (5 mL), and 1N hydrochloric acid (1 mL) was allowed to reflux for 2 hours and was poured into ice water. The resulting precipitate was collected by suction filtration and washed with distilled water and diethyl ether to give 420 mg, 91% yield of a white solid, m.p. 187-189°C.

### f. 1-Cyclopropyl-6-fluoro-8-methoxy-7-[(1S:4S)-5-methyl-2,5-diazabicyclo[2.2.1]hept-2-yl] -1,4-dihydro-4-oxo-3-quinolinecarboxylic acid

A mixture of 1-cyclopropyl-6,7-difluoro-8-methoxy-1,4-dihydro-4-oxo-3-quinolinecarboxylic acid (400 mg, 1.4 mmol), (1S:4S)-2-methyl-2,5-diazabicyclo-[2.2.1]heptane dihydrochloride (300 mg, 1.63 mmol, 1.2 equiv) and 1,8-diazabicyclo[5.4.0]undec-7-ene (710 mL, 4.75 mmol, 3.4 equiv) in anhydrous dimethyl sulfoxide (25 mL) was heated to 75°C for 18 hours. The reaction mixture was cooled to room temperature and poured into distilled water adjusted to pH = 7.4 with saturated aqueous sodium bicarbonate. The aqueous phase was extracted several times with chloroform. The chloroform layer was then extracted with 1N HCl and the acidic aqueous phase was back extracted with chloroform. The aqueous layer was adjusted to pH = 7.4 with saturated aqueous sodium bicarbonate and extracted with chloroform. The chloroform extracts were dried over sodium sulfate, filtered and concentrated in vacuo to a dark oil which was purified by flash chromatography (chloroform/methanol 10:1 v/v) to give 65 mg, 12% yield of a white solid, m.p. 190-212°C with decomposition.

Anal.: Calcd. for C₂₀H₂₂FN₃O₄·0.5H₂O: C, 60.60; H, 5.80; N, 10.60. Found: C, 60.57; H, 5.70; N, 10.59.

## Claims (Claims for the following Contracting State(s): AT, BE, CH, DE, DK, FR, GB, IT, LI, LU, NL, SE)

1. A compound of the formula 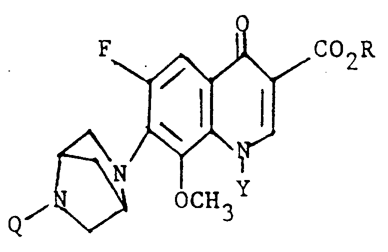 wherein Q is methyl, ethyl, hydroxyethyl or hydrogen; R is hydrogen (C₁-C₃) alkyl, benzyl, (C₁-C₃) alkanoyloxymethyl, (C₁-C₃)alkanoyloxyethyl, benzoyloxymethyl, benzoyloxyethyl, or 5-[(C₁-C₃)-alkyl]-2-oxo-1,3-dioxolen-4-ylmethyl; and Y is cyclopropyl or substituted cyclopropyl, wherein said substituted cyclopropyl is substituted with one to three substituents independently selected from the group consisting of (C₁-C₃)alkyl, halo, hydroxy, or (C₁-C₃)alkoxy; or
a pharmaceutically acceptable salt or hydrate of said compound.

2. A compound according to claim 1, wherein Q is hydrogen.

3. A compound according to claim 1, wherein Q is methyl.

4. A compound according to claim 1, wherein Q is ethyl.

5. A compound according to claim 1, wherein Q is hydroxyethyl.

6. A compound according to claim 1, wherein Y is cyclopropyl.

7. A compound according to claim 1, wherein R is hydrogen.

8. A compound according to claim 1, wherein Q is methyl, Y is cyclopropyl and R is hydrogen.

9. A pharmaceutical composition comprising an antibacterially effective amount of a compound according to claim 1 and a pharmaceutically acceptable carrier.

## Claims (Claims for the following Contracting State(s): ES, GR)

1. A process for making a compound having the formula 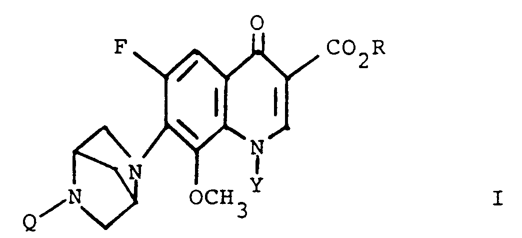 wherein Q is hydrogen; R is hydrogen, (C₁-C₃) alkyl, benzyl, (C₁-C₃) alkanoyloxymethyl, (C₁-C₃) alkanolyoxyethyl, benzoyloxymethyl, benzoyloxyethyl, or 5-[((C₁-C₃)-alkyl]-2-oxo-1,3-dioxolen-4-ylmethyl; and Y is cyclopropyl or substituted cyclopropyl, wherein said substituted cyclopropyl is substituted with one to three substituents independently selected from the group consisting of (C₁-C₃) alkyl, halo, hydroxy, or (C₁-C₃) alkoxy; or a pharmaceutically acceptable salt or hydrate of said compound, comprising reacting a compound having the formula 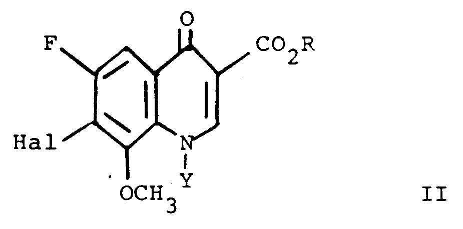 wherein R and Y are defined as above and Hal is chloro, bromo, fluoro or iodo, with a compound of the formula

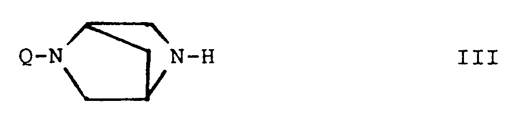

2. A process for making a compound having the formula 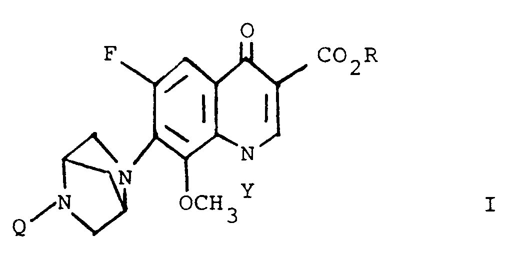 wherein Q is methyl and R and Y are defined as in claim 1, comprising reacting a compound having the formula I, wherein Q is hydrogen and R and Y are defined as in claim 1, with a methylating agent.

3. A process for making a compound having the formula 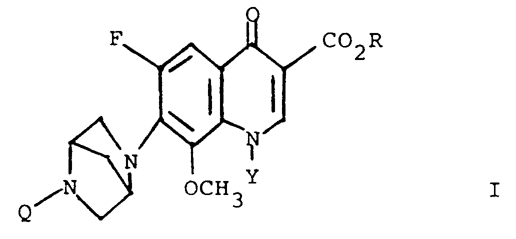 wherein Q is ethyl or hydroxyethyl and R and Y are defined as in claim 1, comprising reacting a compound having the formula I wherein Q is hydrogen, with, respectively, ethyl iodide or 2-bromoethanol.

4. A process according to any of claims 1-3, wherein said process produces a compound of the formula I wherein Y is cyclopropyl.

5. A process according to any of claims 1-3, wherein said process produces a compound of the formula I wherein R is hydrogen.

6. A process according to claim 3, wherein said process produces a compound of the formula I wherein Q is ethyl.

7. A process according to claim 3, wherein said process produces a compound of the formula I wherein Q is hydroxyethyl.

8. A process according to claim 2, wherein said process produces 1-cyclopropyl-6-fluoro-8-methoxy-7-(1S, 4S)-5-methyl-2,5-diazabicyclo[2.2.1]hept-2-yl-1,4-dihydro-4-oxo-3-quinoline carboxylic acid.

## Patentansprüche (Patentansprüche für folgende(n) Vertragsstaat(en): AT, BE, CH, DE, DK, FR, GB, IT, LI, LU, NL, SE)

1. Verbindung der Formel 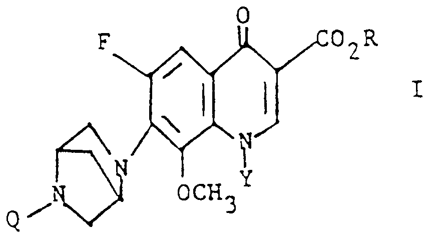 worin Q Methyl, Ethyl, Hydroxyethyl oder Wasserstoff bedeutet; R Wasserstoff, (C₁-C₃)-Alkyl, Benzyl, (C₁-C₃)-Alkanoyloxymethyl, (C₁-C₃)-Alkanoyloxyethyl, Benzoyloxymethyl, Benzoyloxyethyl oder 5-[(C₁-C₃)-Alkyl]-2-oxo-1,3-dioxolen-4-ylmethyl bedeutet; und Y Cyclopropyl oder substituiertes Cyclopropyl bedeutet, worin das substituierte Cyclopropyl mit einem bis drei Substituenten, unabhängig ausgewählt aus der Gruppe, bestehend aus (C₁-C₃)-Alkyl, Halogen, Hydroxy oder (C₁-C₃)-Alkoxy, substituiert ist; oder
ein pharmazeutisch verträgliches Salz oder Hydrat der Verbindung.

2. Verbindung nach Anspruch 1, worin Q Wasserstoff bedeutet.

3. Verbindung nach Anspruch 1, worin Q Methyl bedeutet.

4. Verbindung nach Anspruch 1, worin Q Ethyl bedeutet.

5. Verbindung nach Anspruch 1, worin Q Hydroxyethyl bedeutet.

6. Verbindung nach Anspruch 1, worin Y Cyclopropyl bedeutet.

7. Verbindung nach Anspruch 1, worin R Wasserstoff bedeutet.

8. Verbindung nach Anspruch 1, worin Q Methyl bedeutet, Y Cyclopropyl bedeutet und R Wasserstoff bedeutet.

9. Pharmazeutische Zusammensetzung, umfassend eine antibakteriell wirksame Menge einer Verbindung nach Anspruch 1 und einen pharmazeutischen Träger.

## Patentansprüche (Patentansprüche für folgende(n) Vertragsstaat(en): ES, GR)

1. Verfahren zur Herstellung einer Verbindung der Formel 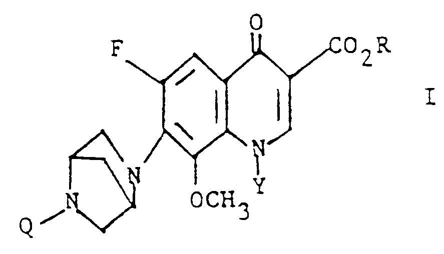 worin Q Wasserstoff bedeutet; R Wasserstoff, (C₁-C₃)-Alkyl, Benzyl, (C₁-C₃)-Alkanoyloxymethyl, (C₁-C₃)-Alkanoyloxyethyl, Benzoyloxymethyl, Benzoyloxyethyl oder 5-[(C₁-C₃)-Alkyl]-2-oxo-1,3-dioxolen-4-ylmethyl bedeutet; und Y Cyclopropyl oder substituiertes Cyclopropyl bedeutet, worin das substituierte Cyclopropyl mit einem bis drei Substituenten, unabhängig ausgewählt aus der Gruppe, bestehend aus (C₁-C₃)-Alkyl, Halogen, Hydroxy oder (C₁-C₃)-Alkoxy, substituiert ist; oder
eines pharmazeutisch verträglichen Salzes oder Hydrates der Verbindung, umfassend Umsetzen einer Verbindung der Formel 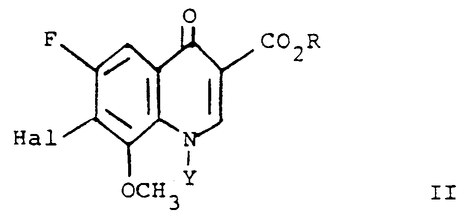 worin R und Y wie vorstehend definiert sind und Hal Chlor, Brom, Fluor oder Jod bedeutet, mit einer Verbindung der Formel

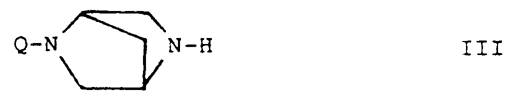

2. Verfahren zur Herstellung einer Verbindung der Formel 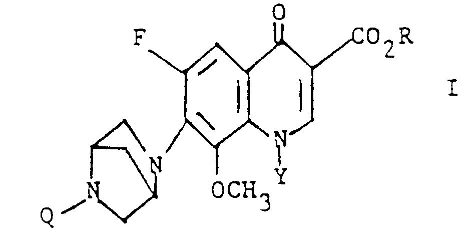 worin Q Methyl bedeutet und R und Y wie in Anspruch 1 definiert sind, umfassend Umsetzen einer Verbindung der Formel I, worin Q Wasserstoff bedeutet und R und Y wie in Anspruch 1 definiert sind, mit einem Methylierungsmittel.

3. Verfahren zur Herstellung einer Verbindung der Formel 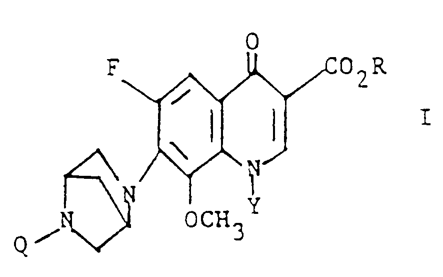 worin Q Ethyl oder Hydroxyethyl bedeutet, und R und Y wie in Anspruch 1 definiert sind, umfassend Umsetzen einer Verbindung der Formel I, worin Q Wasserstoff bedeutet, mit Ethyljodid bzw. 2-Bromethanol.

4. Verfahren nach einem der Ansprüche 1 bis 3, wobei das Verfahren eine Verbindung der Formel I liefert, worin Y Cyclopropyl ist.

5. Verfahren nach einem der Ansprüche 1 bis 3, wobei das Verfahren eine Verbindung der Formel I liefert, worin R Wasserstoff bedeutet.

6. Verfahren nach Anspruch 3, wobei das Verfahren eine Verbindung der Formel I liefert, worin Q Ethyl bedeutet.

7. Verfahren nach Anspruch 3, wobei das Verfahren eine Verbindung der Formel I liefert, worin Q Hydroxyethyl bedeutet.

8. Verfahren nach Anspruch 2, wobei das Verfahren 1-Cyclopropyl-6-fluor-8-methoxy-7-(1S,4S)-5-methyl-2,5-diazabicyclo[2.2.1]hept-2-yl-1,4-dihydro-4-oxo-3-chinolincarbonsäure liefert.

## Revendications (Revendications pour l'(les) Etat(s) contractant(s) suivant(s): AT, BE, CH, DE, DK, FR, GB, IT, LI, LU, NL, SE)

1. Composé de formule 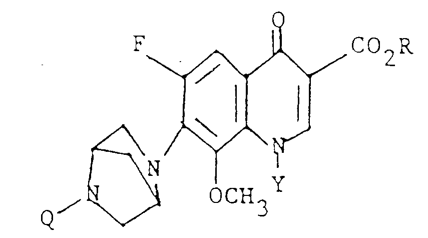 dans laquelle Q est un groupe méthyle, éthyle, hydroxyéthyle ou de l'hydrogène ; R est de l'hydrogène, un groupe alkyle en C₁ à C₃, benzyle, (alcanoyloxy en C₁ à C₃)-méthyle, (alcanoyloxy en C₁ à C₃)-éthyle, benzoyloxyméthyle, benzoyloxyéthyle ou 5-(alkyle en C₁ à C₃)-2-oxo-1,3-dioxolén-4-yl-méthyle ; et Y est un groupe cyclopropyle ou cyclopropyle substitué, ce groupe cyclopropyle substitué portant 1 à 3 substituants choisis indépendamment dans le groupe des radicaux alkyle en C₁ à C₃, halogéno, hydroxy ou alkoxy en C₁ à C₃ ; ou
un sel pharmaceutiquement acceptable ou un hydrate de ce composé.

2. Composé suivant la revendication 1, dans lequel Q est l'hydrogène.

3. Composé suivant la revendication 1, dans lequel Q est un groupe méthyle.

4. Composé suivant la revendication 1, dans lequel Q est un groupe éthyle.

5. Composé suivant la revendication 1, dans lequel Q est un groupe hydroxyéthyle.

6. Composé suivant la revendication 1, dans lequel Y est un groupe cyclopropyle.

7. Composé suivant la revendication 1, dans lequel R est de l'hydrogène.

8. Composé suivant la revendication 1, dans lequel Q est un groupe méthyle, Y est un groupe cyclopropyle et R est de l'hydrogène.

9. Composition pharmaceutique comprenant une quantité à effet antibactérien d'un composé suivant la revendication 1 et un support acceptable du point de vue pharmaceutique.

## Revendications (Revendications pour l'(les) Etat(s) contractant(s) suivant(s): ES, GR)

1. Procédé de production d'un composé répondant à la formule 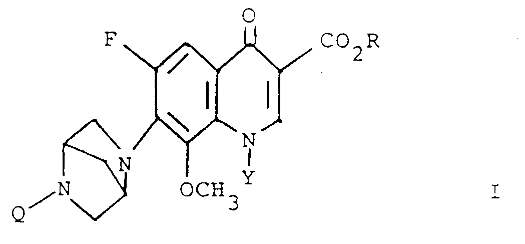 dans laquelle Q est l'hydrogène ; R est de l'hydrogène, un groupe alkyle en C₁ à C₃, benzyle, (alcanoyloxy en C₁ à C₃)-méthyle, (alcanoyloxy en C₁ à C₃)-éthyle, benzoyloxyméthyle, benzoyloxyéthyle ou 5-(alkyle en C₁ à C₃)-2-oxo-1,3-dioxolén-4-ylméthyle ; et Y est un groupe cyclopropyle ou cyclopropyle substitué, ce groupe cyclopropyle substitué portant 1 à 3 substituants choisis indépendamment dans le groupe des radicaux alkyle en C₁ à C₃, halogéno, hydroxy ou alkoxy en C₁ à C₃ ; ou d'un sel pharmaceutiquement acceptable ou d'un hydrate de ce composé, comprenant la réaction d'un composé de formule 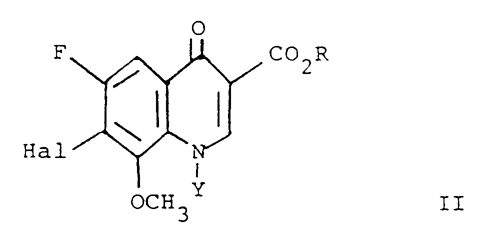 dans laquelle R et Y sont tels que définis ci-dessus et Hal est un radical chloro, bromo, fluoro ou iodo, avec un composé de formule

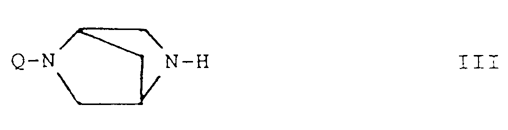

2. Procédé de production d'un composé de formule 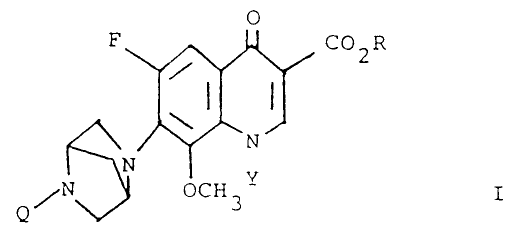 dans laquelle Q est un groupe méthyle et R et Y sont tels que définis dans la revendication 1, comprenant la réaction d'un composé de formule I dans laquelle Q est de l'hydrogène et R et Y sont tels que définis dans la revendication 1, avec un agent de méthylation.

3. Procédé de production d'un composé de formule 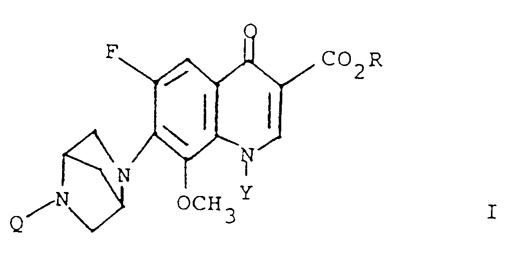 dans laquelle Q est un groupe éthyle ou hydroxyéthyle et R et Y sont tels que définis dans la revendication 1, comprenant la réaction d'un composé de formule I dans laquelle Q est de l'hydrogène avec, respectivement, l'iodure d'éthyle ou le 2-brométhanol.

4. Procédé suivant l'une quelconque des revendications 1 à 3, qui produit un composé de formule I dans laquelle Y est un groupe cyclopropyle.

5. Procédé suivant l'une quelconque des revendications 1 à 3, qui produit un composé de formule I dans laquelle R est de l'hydrogène.

6. Procédé suivant la revendication 3, qui produit un composé de formule I dans laquelle Q est un groupe éthyle.

7. Procédé suivant la revendication 3, qui produit un composé de formule I dans laquelle Q est un groupe hydroxyéthyle.

8. Procédé suivant la revendication 2, qui produit l'acide 1-cyclopropyl-6-fluoro-8-méthoxy-7-(1S, 4S)-5-méthyl-2,5-diazabicyclo[2.2.1]hept-2-yl-1,4-dihydro-4-oxo-3-quinoléine-carboxylique.
